**Europäisches Patentamt**

**European Patent Office**

**Office européen des brevets**

(19)

(11) Veröffentlichungsnummer: **0 246 518**
A2

# (12) EUROPÄISCHE PATENTANMELDUNG

(21) Anmeldenummer: **87106728.6**

(22) Anmeldetag: **08.05.87**

(51) Int. Cl.³: **C 12 N 5/00**

Ein Antrag gemäss Regel 88 EPÜ auf Berichtigung der Seite 6 liegt vor. Über diesen Antrag wird im Laufe des Verfahrens von der Prüfungsabteilung eine Entscheidung getroffen werden.

(30) Priorität: **23.05.86 DE 3617399**

(43) Veröffentlichungstag der Anmeldung:
**25.11.87 Patentblatt 87/48**

(84) Benannte Vertragsstaaten:
**AT BE CH DE ES FR GB GR IT LI LU NL SE**

(71) Anmelder: **Siegel, Rolf, Dr.**
**Versbacher Strasse 180**
**D-8700 Würzburg(DE)**

(72) Erfinder: **Siegel, Rolf, Dr.**
**Versbacher Strasse 180**
**D-8700 Würzburg(DE)**

(74) Vertreter: **von Füner, Alexander, Dr. et al,**
**Patentanwälte v. Füner, Ebbinghaus, Finck Mariahilfplatz 2 & 3**
**D-8000 München 90(DE)**

(54) Unterlage für Zellkulturen.

(57) Es wird eine Unterlage für Zellkulturen mit basalmembranähnlichen Eigenschaften beschrieben: sie ist im μm-Bereich dünn, ist mechanisch stabil und flexibel, weist auf ihrer Oberfläche Kohlenhydrate und/oder Kohlenhydratgruppenketten und/oder Proteine und/oder amphiphile Substanzen auf und ist für Ionen und Proteine permeabel.

EP 0 246 518 A2

v. FÜNER     EBBINGHAUS     FINCK

PATENTANWÄLTE     EUROPEAN PATENT ATTORNEYS

MARIAHILFPLATZ 2 & 3, MÜNCHEN 90
POSTADRESSE: POSTFACH 95 01 60, D-8000 MÜNCHEN 95

0246518

Rolf Siegel

8. Mai 1987
EPAA-34962.2

## UNTERLAGE FÜR ZELLKULTUREN

Bei der Erfindung handelt es sich um Unterlagen für Zell-
und Gewebekulturen in vitro. Diese Unterlagen, auch Substrate genannt, sind ausbildungsgemäß als Zellkulturröhrchen, Zellkulturschalen, "microcarrier beads" und "Roux
bottles" bekannt.

Die im folgenden beschriebenen Substrate eignen sich bevorzugt für Zellkulturen von zum vektoriellen Stofftransport
befähigten Zellen: durch geeignete Anordnung dieser erfindungsgemäßen Substrate ist es leicht möglich, Syntheseprodukte in konzentrierter Form zu gewinnen oder Stoffgradienten aufzubauen.

Beispiele für zum vektoriellen Stofftransport befähigte
Zellen sind Epithelzellen, beispielsweise aus Niere, Gastrointestinaltrakt und Drüsengewebe. Auch Anordnungen von zusammenhängenden Zellverbänden, wie Schwämme (Spongien)
oder Hohltiere (Coelenterata) oder Anordnungen von normalerweise einzelligen Organismen werden hierunter subsumiert, so daß in diesem Zusammenhang der Begriff Zellkultur umfassender als bisher verstanden wird.

In der DE-PS 34 40 557 wird eine Unterlage für das Zellwachstum in vitro beschrieben. Es handelt sich hierbei
um mit Lipoiden beschichtete hydrophobe Materialien. So
wird nach Ausbildung eines dichten Zellrasens auf einem
derart beschichteten Filter durch geeignete Manipulation
ein stoffwechselenergieabhängiger Stoffgradient gewonnen
und aufrechterhalten. Da nur die Zellen, die die Filterporen verschließen, für den ungehinderten transzellulären
Stofftransport zur Verfügung stehen, sind derartige Materialien für diese Konzentrationszwecke nur bedingt geeignet.
Der überwiegende Teil der auf der Filteroberfläche haftenden Zellen (ca. 85%) trägt nicht zur Schaffung und Aufrechterhaltung eines Stoffgradienten bei, was letztendlich durch die dichte Struktur des Filtermaterials bedingt
ist.

Die im folgenden beschriebene Unterlage soll es ermöglichen,
Syntheseprodukte von Zellen in vitro in konzentrierter Form
zu gewinnen bzw. Stoffwechselleistungen von Zellen in Zellkultur dadurch optimal auszunutzen, daß die gesamte Oberfläche der Unterlage hierfür zur Verfügung steht.

Erfindungsgemäß werden als Ausgangsmaterialien für die
Unterlagen im μm-Bereich liegende dünne, mechanisch stabile, flexible, hydrophile, für Ionen und Proteine permeable
Materialien, die mindestens eine reaktive (funktionelle)
Gruppe auf ihrer Oberfläche aufweisen, verwendet. Solche
Materialien stehen beispielsweise als Dialysiermembranen
aus Cellulose (beispielsweise mit zwischen 3.500 und
14.000 Dalton stufenweise frei wählbaren "molecular weight
cutoff") oder aber auch in Form von Cellophan-Folien zur
Verfügung.

Die Oberflächen dieser Ausgangsmaterialien werden nun
ihrerseits mit natürlichen und/oder synthetischen biologisch aktiven Substanzen, wie Kohlenhydraten und/oder
Kohlenhydratgruppenketten und/oder Proteinen und/oder amphiphilen Substanzen als Reinsubstanzen oder mengenmäßig definierte Mischungen so umgesetzt, daß sich zwischen den biologisch aktiven Substanzen und der Materialoberfläche im physiologischen Milieu stabile Bindungen ausbilden.

Nach der Umsetzung der Ausgangsmaterialien mit Kohlenhydraten und/oder Kohlenhydratgruppenketten und/oder Proteinen und/oder amphiphilen Substanzen stellen diese die unmittelbare Oberfläche der Unterlage dar und bieten "Ankermöglichkeiten" für Zellen (siehe auch H. Rauvala, Trends in Biochemistry Science, 7, 323 - 325 (1983)).

Oben erwähnte synthetische bzw. natürliche biologische Substanzen sind bekannt, sind in reiner Form leicht erhältlich, bzw. können leicht gewonnen werden.

Ausgestaltungsgemäß werden natürliche biologisch aktive Substanzen, die den hydrophilen Anteil ("Kopf") von natürlichen Lipoiden bilden, eingesetzt. Diese natürlichen Lipoide können problemlos als Lipoid-Extrakt aus tierischen oder pflanzlichen Materialien gewonnen werden und umfassen je nach Aufarbeitung unterschiedliche Mengen an Glycerin enthaltende Lipide wie Mono-, Di-, Triacylglycerine, Glyceryläther (Alkyloxydiglyceride), Glycosylmono- und diacylglycerine, Phospholipide (Phosphatide), Plasmalogene (Acetalphosphatide), Sphingosin enthaltende Lipide wie Sphingomyeline, Ceramide, neutrale Glycosphingolipide (Cerebroside), Sialoglycosphingolipide (Ganglioside), Sulfoglycosphingolipide (Sulfatide), komplexe Lipide wie Lipoproteine, Proteolipide und Lipopolysaccharide. Wegen des hohen Anteils an (verzweigten) Kohlenhydratgruppenketten wird die Extraktion aus Erythrozyten, Synovia, Hirn und Plazenten bevorzugt.

Die Gewinnung der die biologisch aktiven Substanzen darstellenden Kohlenhydrate und/oder Kohlenhydratgruppenketten und/oder Proteine erfolgt seinerseits ausgestaltungsgemäß durch enzymatische Hydrolysierung oben erwähnter natürlicher Lipoide, so daß Substanzen wie Fucose, Mannose, Galaktose, Glucose, N-Acetylgalactosamin, N-Acylneuraminsäure und Glykosaminglykane leicht erhältlich sind, Verbindungen von denen bekannt sind, daß sie ein geordnetes Zellwachstum begünstigen. Einfache Substanzen, wie einfache Zucker, Steroide, Terpene, Cholesterin, Vitamine und Alkohole werden direkt zur Beschichtung eingesetzt.

Die erwähnten biologisch aktiven Substanzen sind physikalisch-chemisch, z.B. hinsichtlich ihres Ionisierungsgrades und ihrer Molekülradien gut definiert. So ist es beispielsweise einfach, Substratoberflächen mit positiven oder negativen Oberflächenladungen herzustellen: setzt man z.B. das Hydrolyseprodukt von Phosphatidylserin und/oder Cardiolipin als biologisch aktive Substanzen ein, so erhält man Substratoberflächen mit negativer Ladung bei physiologischen pH. Natürlich müssen die jeweiligen prozentualen Anteile der eingesetzten biologisch aktiven Substanzen hinsichtlich optimaler Zellhaftung und Zellwachstum von Zellinie zu Zellinie experimentell ermittelt werden. Dies ist aber ohne weiteres möglich.

Die unter physiologischen Bedingungen stabile Beschichtung der Oberflächen der Ausgangsmaterialien mit den erwähnten biologisch aktiven Substanzen erfolgt durch Inkubation. Unter geeigneten Reaktionsbedingungen kommt es im wässrigen Milieu zur Ausbildung von kovalenten und/oder adsorptiven Bindungen zwischen reaktiven Gruppen von Ausgangsmaterial und eingesetzten biologisch aktiven Substanzen. Zur Verkürzung der Inkubationszeit ist es ohne weiteres möglich, das Ausgangsmaterial vorher mit BrCN zu aktivieren.

Nach gründlichem Waschen mit geeigneten Puffern kann nunmehr das erfindungsgemäße Substrat zur Anzüchtung von

- 5 -

Zellen in bekannter Art und Weise eingesetzt werden.

<u>Beispiel</u>

Als Ausgangsmaterial für die erfindungsgemäße Unterlage
wird eine Dialysiermembran aus Cellulose ("molecular
weight cutoff" 6.000 - 8.000 Dalton) verwendet.

Die zur Beschichtung des Filters erforderlichen biologisch
aktiven Substanzen werden zunächst als Gesamtlipid-Extrakt aus Vollblut - in Anlehnung an Folch et al (J.
Folch, M. Lees and G. H. Stanley: J. Biol. Chem. 226, 497
(1957)) - gewonnen: ein Teil EDTA-Blut wird mit 9 Teilen
eines Chloroform/Methanol-Gemisches versetzt und die resultierende Lipoid-Phase wird anschließend zur Trockene
eingeengt. Danach werden die Lipoide in einer mit einer
mit Ringer-Lösung gesättigten Butanol-Lösung resuspendiert (1 Volumenteil). Diese Suspension wird nun für
ca. 24 Stunden bei 28$^{\circ}$C mit einer Enzym-Lösung (1 Volumenteil, welche Phospholipase C (EC 3. 1. 4. 3),
Sphingomyelinase (EC 3. 1. 4. 12) und Lipase (EC 3. 1.
1. 3) enthält, unter ständiger rührender Bewegung inkubiert.

Die Trennung der biologisch aktiven hydrophilen Substanzen von den Aliphaten und nicht hydrolisierten Lipoiden
erfolgt durch Zugabe von 0,1 Volumenteil Chloroform. Die
obere wässrige, die biologisch aktiven Substanzen enthaltende Phase wird nach säulenchromatographischer Abtrennung der Enzyme für die erfindungsgemäße Beschichtung
des Ausgangsmaterials weiterverwendet.

Das Ausgangsmaterial wird nun mit der die biologisch aktiven Substanzen enthaltenden Lösung solange bei 28$^{\circ}$C inkubiert, bis das Lösungsmittel vollständig verdampft und
die Dialysiermembran trocken ist.

Die derart beschichtete Membran wird nunmehr gründlich mit Ringerlösung gewaschen, vorsichtig getrocknet und in einen Filterhalter eingespannt und dampfsterilisiert. Danach wird sie in ein verschließbares Gefäß mit Möglichkeiten zur Luft- und Kohlendioxidzuleitung bzw. zur kontinuierlichen Absaugung- und Zuführung von Nährmedium derart gestellt, daß die erfindungsgemäß beschichtete Membran von Nährmedium auf beiden Seiten völlig bedeckt ist.

Nach einer ca. 24 Stunden dauernden Äquilibrierungsperiode wird der Filter aus dem Gefäß genommen und in bekannter Art und Weise mit einer etablierten Zellinie aus Hamsternieren (BHK) auf einer Seite beimpft und anschließend in dem nährmediumenthaltenden Gefäß unter den üblichen Bedingungen inkubiert.

Nach ca. drei bis vier Tagen hat sich ein gleichmäßiger Epithelzellrasen auf der beschichteten Membranoberfläche ausgebildet und kann gemäß der in der DE-PS 34 40 557 beschriebenen Methode zum induzierten, stoffwechselaktiven vektoriellen Stofftransport weiterverwendet werden.

## Patentansprüche

1. Unterlagen für Zellkulturen, wobei es sich um dünne, im $\mu$m-Bereich liegende, mechanisch stabile, flexible, für Ionen und Proteine permeable Materialien, die mindestens eine Art reaktiver Gruppe auf ihrer Oberfläche aufweisen, handelt, die ihrerseits mit biologisch aktiven Substanzen als Reinsubstanzen oder mengenmäßig definierten Mischungen derart beschichtet sind, daß die entstehenden Bindungen zwischen Materialoberfläche und biologisch aktiven Substanzen im physiologischen Milieu stabil sind, dadurch g e k e n n z e i c h n e t , daß als dünne, im $\mu$m-Bereich liegende, mechanisch stabile, flexible, für Ionen und Proteine permeable Materialien, die mindestens eine Art reaktiver Gruppe auf ihrer Oberfläche aufweisen, Dialysiermembranen aus Cellulose oder Cellophan®-Folien eingesetzt sind.

2. Unterlagen für Zellkulturen nach Anspruch 1, dadurch g e k e n n z e i c h n e t , daß als biologisch aktive Substanzen solche, die durch enzymatische Hydrolyse aus natürlichen Lipoiden gewonnen werden, eingesetzt sind.

**v. FÜNER · EBBINGHAUS FINCK**

PATENTANWÄLTE EUROPEAN PATENT ATTORNEYS

MARIAHILFPLATZ 2 & 3, MÜNCHEN 90

POSTADRESSE: POSTFACH 95 01 60, D-8000 MÜNCHEN 95

0246518

KARL LUDWIG SCHIFF (1964 – 1978)
DIPL. CHEM. DR. ALEXANDER v. FÜNER
DIPL. ING. DIETER EBBINGHAUS
DR. ING. DIETER FINCK

TELEFON (089) 48 20 54
TELEX 5-23 565 AURO D
TELEFAX III - II - AUTOM. (089) 48 20 58
TELEGRAMME AUROMARCPAT MÜNCHEN

KONTEN / ACCOUNTS:
BAYER. VEREINSBANK MÜNCHEN 626 0004
US-$ CURRENCY ACCOUNT 806 227 102
SWIFT-ADRESSE: BVBE DE MM

Europäisches Patentamt

8000 München

EPA·EPO·OEB
MÜNCHEN
Empfang bestätigt
Receipt acknowledged
Accuse reception

EPA EPO-OEB
DG 1
R...

2 6 MAI 1987

18. Mai 1987

Betr.: Europäische Patentanmeldung Nr. 87 106 728.6
Dr. Rolf SIEGEL
Unsere Akte: EPAA-34962.2

In der Anlage wird eine neue Seite 6 mit der Bitte eingereicht, die durch * gekennzeichnete Verbesserung zu berücksichtigen.

Dr. A.v.Füner

Anlage: Seite 6, 3-fach

vF/fg

mit Ringerlösung gewaschen, vorsichtig getrocknet und in einen Filterhalter eingespannt und dampfsterilisiert. Danach wird sie in ein verschließbares Gefäß mit Möglichkeiten zur Luft- und Kohlendioxidzuleitung bzw. zur kontinuierlichen Absaugung- und Zuführung von Nährmedium derart gestellt, daß die erfindungsgemäß beschichtete Membran von Nährmedium auf beiden Seiten völlig bedeckt ist.

Nach einer ca. 24 Stunden dauernden Äquilibrierungsperiode wird der Filter aus dem Gefäß genommen und in bekannter Art und Weise mit einer etablierten Zellinie aus ~~Madin Darby canine (MDCK)~~ * auf einer Seite beimpft und anschließend in dem nährmediumenthaltenden Gefäß unter den üblichen Bedingungen inkubiert.

Nach ca. drei bis vier Tagen hat sich ein gleichmäßiger Epithelzellrasen auf der beschichteten Membranoberfläche ausgebildet und kann gemäß der in der DE-PS 34 40 557 beschriebenen Methode zum induzierten, stoffwechselaktiven vektoriellen Stofftransport weiterverwendet werden.

\* Maden Darby canine kidney (MDCK)